# FASCICULE DE BREVET EUROPEEN

(11) **EP 4 517 325 B1**
(45) Date de publication et mention de la délivrance du brevet: **29.04.2026**
(21) Numéro de dépôt: 24197096.1
(22) Date de dépôt: 28.08.2024
(51) Int. Cl.: G01N 33/543, G01N 33/558

(54) **DISPOSITIF DE DIAGNOSTIC POUR DÉTECTER ET/OU IDENTIFIER UN ANALYTE CIBLE AU DÉPART D'UN ÉCHANTILLON LIQUIDE**
DIAGNOSEVORRICHTUNG ZUM NACHWEIS UND/ODER IDENTIFIZIEREN EINES ZIELANALYTEN AUS EINER FLÜSSIGKEITPROBE
DIAGNOSTIC DEVICE FOR DETECTING AND/OR IDENTIFYING A TARGET ANALYTE FROM A LIQUID SAMPLE

(30) Priorité: 29.08.2023 BE 202305712
(43) Date de publication de la demande: 05.03.2025
(73) Titulaire: Actifar, 1435 Mont Saint Guibert (BE)
(72) Inventeur: Maerevoet, Béatrice, 1300 Limal (BE); Naessens, Olivier, 5310 Eghezée (BE)
(74) Mandataire: Gevers Patents

(56) Documents cités:
- WO-A1-2019/113118
- DE-U1- 202022 106 585
- US-A1- 2022 137 043

## Description

La présente invention porte sur un dispositif de diagnostic pour détecter et/ou identifier un analyte cible dans un échantillon liquide. En particulier, la présente invention porte sur un test de grossesse basé sur la détection de l'hormone hCG.

Un dispositif selon la présente invention peut permettre la détection et/ou l'identification de tout type d'analyte, souvent appelé analyte d'intérêt ou analyte cible. Par exemple, un dispositif selon la présente invention peut permettre de détecter et/ou d'identifier un analyte d'intérêt étant une toxine, un composé organique, une protéine, un peptide, un microorganisme, une bactérie, un virus, un acide aminé, un acide nucléique, un glucide, une hormone, un stéroïde, une vitamine, un médicament, un polluant, un pesticide, une substance antigénique, un haptène ou encore un anticorps.

De nombreux dispositifs de diagnostic sont disponibles dont certains sont composés d'une coque (ou support) en plastique contenant une bandelette réactive (ou languette de détection) synthétique en un matériau plastique, typiquement en nitrocellulose. Une approche plus écologique visant à réduire considérablement les déchets plastiques générés par l'utilisation de tels dispositifs de diagnostic a conduit au développement de dispositifs de diagnostic composés d'une coque (ou support) en papier ou en carton contenant une bandelette réactive (ou languette de détection) en un matériau plastique, typiquement en nitrocellulose. Les membranes ou bandelette en nitrocellulose sont synthétiques et non biodégradables.

En ce qui concerne les tests de grossesse, ces dispositifs sont généralement basés sur un dosage immunologique biochimique, testant la présence de niveaux élevés de l'hormone gonadotrophine chorionique humaine (hCG) dans les fluides corporels, tels que l'urine. L'hormone hCG est générée au début de la grossesse et constitue ainsi un analyte indicateur très utile. L'immunodosage est généralement basé sur l'utilisation d'anticorps anti-hCG et d'antigènes adaptés pour se coupler à de tels anticorps, au niveau de sites de liaison sur l'anticorps.

Classiquement, les dispositifs de test de grossesse reposent sur une utilisation d'un échantillon d'urine s'infiltrant par capillarité le long d'une bandelette réactive. Un réservoir d'anticorps anti-hCG lié à un matériau particulaire coloré est rencontré en premier. Si l'hCG est présente, les anticorps s'y couplent, formant un complexe qui est emporté le long de la bandelette réactive. Plus loin le long de la bandelette réactive se trouve une zone linéaire transversale comportant un réactif ancré auquel le complexe hCG/anticorps se liera. Aussi, si de l'hCG est présente dans l'urine, le complexe hCG/anticorps et le matériau coloré associé s'accumuleront dans cette zone, formant une ligne colorée visible sur la bandelette réactive.

Le brevet DE202022106585 divulgue un test de de grossesse comprenant une plaque de support sur laquelle est fixée au moins une bandelette de test dotée d'une section de réception d'échantillon et d'une section de test. Ce dispositif présente une section de détachement qui, une fois retirée ou soulevée selon la ligne de perforation, donne accès à la section de réception d'échantillon d'au moins une bandelette réactive.

Malheureusement, les dispositifs de diagnostic actuels, par exemple les tests de grossesse actuels, restent peu écologique. En ce sens, même s'ils présentent une coque (ou support) en papier ou en carton, les tests de grossesse actuels constituent malgré tout des déchets non facilement recyclables dès lors qu'ils comprennent d'une part une bandelette réactive en un matériau plastique et d'autre part une coque (ou support) en papier ou en carton. Après utilisation de tels dispositifs, l'utilisateur se retrouve face à un déchet comprenant deux matériaux ne se recyclant pas / ne se traitant pas / ne s'éliminant pas de la même façon puisque les matériaux plastiques et les matériaux de type papier ou carton suivent des filières de recyclage, de traitement ou d'élimination bien distinctes.

Pour répondre aux attentes des consommateurs étant de plus en plus demandeurs de produits écologiques (produits verts) et/ou pour réduire la pollution environnementale et/ou pour répondre à des obligations règlementaires, il existe donc aujourd'hui un réel besoin de procurer des dispositifs de diagnostic dont un recyclage, un traitement ou une élimination est possible et aisé.

Pour résoudre ces problèmes, il est prévu suivant l'invention, un dispositif de diagnostic pour détecter et/ou identifier un analyte cible dans un échantillon liquide, comprenant :
- un support présentant une face supérieure et une face inférieure, et
- au moins une bandelette réactive renfermée à l'intérieur dudit support entre ladite face supérieure et ladite face inférieure, ladite au moins une bandelette réactive comprenant une zone test et des réactifs permettant de détecter et/ou d'identifier ledit analyte cible,

ladite face supérieure dudit support comprenant :
   - au moins une ouverture agencée pour donner accès à une zone de dépôt dudit échantillon liquide, ladite zone de dépôt étant en communication fluidique avec ladite zone test de ladite au moins une bandelette réactive, et
   - une fenêtre de lecture s'ouvrant sur ladite zone test de ladite au moins une bandelette réactive où a lieu la détection et/ou l'identification dudit analyte cible,
ledit dispositif de diagnostic étant caractérisé en ce que ledit support présente au moins une zone de rupture conçue pour donner accès à un utilisateur à une extrémité libre de ladite au moins une bandelette réactive.

Par les termes « ledit support présente au moins une zone de rupture conçue pour donner accès à un utilisateur à une extrémité libre de ladite au moins une bandelette réactive », il est entendu au sens de la présente invention que le support comprend un ou des moyen(s) permettant de le casser et/ou de le rompre au moins partiellement. En particulier, selon la présente invention, la « zone de rupture » peut permettre de casser et/ou de rompre la face supérieure du support ou la face inférieure du support ou à la fois la face supérieure et la face inférieure du support, de telle sorte à donner accès à un utilisateur à une extrémité libre d'au moins une bandelette réactive. Ledit support selon la présente invention présente donc au moins une zone de rupture agencée pour donner accès à un utilisateur à une extrémité libre d'au moins une bandelette réactive

Par les termes « extrémité libre de ladite au moins une bandelette réactive», il est entendu, au sens de la présente invention, une extrémité d'au moins une bandelette réactive qui est non fixée au support et qui est directement accessible pour un utilisateur suite à une rupture de la zone de rupture, c'est-à-dire lorsque la zone de rupture a été utilisée afin de casser et/ou de rompre le support. Selon l'invention, l'extrémité libre d'au moins une bandelette réactive n'est donc pas fixée / attachée / accrochée au support par un quelconque moyen. L'extrémité libre n'est pas non plus bloquée dans le support de telle sorte qu'elle peut en être aisément saisie pour en être séparée.

Avec un tel dispositif de diagnostic selon l'invention présentant une zone rupture conçue pour / agencée pour donner accès à un utilisateur à une extrémité libre de ladite au moins une bandelette réactive, il est aisé de séparer au moins une bandelette réactive (par exemple en un matériau plastique) du support (par exemple en papier ou en carton). Par conséquent, un utilisateur peut facilement diriger de façon distincte, vers la filière de recyclage / de traitement / d'élimination adéquate, au moins une bandelette réactive en un matériau plastique et le support en papier ou en carton. Ceci permet entre autre d'éviter l'incinération du dispositif de diagnostic. Un dispositif de diagnostic selon l'invention est donc écologique, répond aux attentes des consommateurs en termes de réduction de l'empreinte écologique et va dans le sens des obligations règlementaires présentes et à venir. Tout cela permet le recyclage et la gestion des déchets, car chaque composant peut être traité individuellement de manière appropriée.

Au sens de la présente invention, le terme « échantillon » fait référence à tout ce qui peut contenir un analyte pour lequel une détection et/ou une identification d'analyte est souhaitée. L'échantillon peut être un échantillon biologique, tel qu'un échantillon de fluide biologique. Des exemples de fluides biologiques comprennent l'urine, le sang, le plasma, le sérum, la salive, le sperme, les selles, les expectorations, le liquide céphalo-rachidien, les larmes, le mucus, le liquide amniotique ou similaire. De tels échantillons peuvent être des échantillons humains, animaux ou fabriqués par l'homme.

Au sens de la présente invention, les termes « échantillon fluide » ou « échantillon liquide » font référence à un matériau suspecté de contenir l'analyte d'intérêt, lequel matériau présente une fluidité suffisante pour s'écouler à travers un dispositif de diagnostic selon l'invention.

L'échantillon de fluide ou échantillon liquide peut être utilisé tel qu'obtenu directement à la source ou suite à un prétraitement de manière à modifier son caractère. Le prétraitement éventuel de l'échantillon peut impliquer la préparation de plasma à partir de sang, la dilution de fluides visqueux, la filtration, la distillation, la séparation, la concentration, l'inactivation des composants interférents et l'ajout de réactifs.

Typiquement, l'échantillon est une solution aqueuse ou un fluide biologique. L'échantillon liquide peut provenir de n'importe quelle source, telle qu'un liquide physiologique, y compris le sang, le sérum, le plasma, la salive, les expectorations, le liquide de la lentille oculaire, la sueur, l'urine, le lait, le liquide d'ascite, le mucus, le liquide synovial, le liquide péritonéal, les exsudats transdermiques, les exsudats pharyngés, le lavage bronchoalvéolaire, les aspirations trachéales, le liquide céphalo-rachidien, le sperme, la glaire cervicale, les sécrétions vaginales ou urétrales ou encore le liquide amniotique.

Un matériau solide suspecté de contenir l'analyte peut être utilisé comme échantillon après modification pour former un milieu liquide ou pour libérer l'analyte.

Au sens de la présente invention, le terme « biodégradable » fait référence à un matériau qui est capable d'être décomposé par des bactéries ou d'autres organismes vivants, des processus naturels ou d'autres agents ou moyens biologiques. Par exemple, au sens de la présente invention, peuvent être considérés comme étant des matériaux biodégradables le cartonet le papier.

Au sens de la présente invention, les termes « communication fluidique » font référence à la disposition ou à l'agencement d'un ou de plusieurs matériaux de telle sorte que le fluide est capable de s'écouler à travers le matériau ou de s'écouler entre les matériaux par capillarité, écoulement absorbant ou écoulement axial. Un matériau peut être en « communication fluidique » avec un autre matériau indépendamment de la présence de fluide s'il offre la capacité de permettre l'écoulement de fluide entre des matériaux lorsqu'un fluide est présent.

Au sens de la présente invention, les termes « bandelette réactive » ou « languette de détection » font référence à une partie/à un composant du dispositif de diagnostic comprenant des réactifs et une zone test en communication fluidique avec une zone de dépôt/zone de réception d'échantillon.

Au sens de la présente invention, les termes « zone de dépôt d'échantillon » (également appelée « zone d'échantillon ») font référence à une zone où un échantillon est mis en contact avec le dispositif de diagnostic. Selon un mode de réalisation suivant l'invention, la zone de dépôt d'échantillon est prévue pour accueillir un échantillon de fluide (ou échantillon liquide) qui peut contenir un analyte d'intérêt. Dans un autre mode de réalisation, la zone de dépôt d'échantillon est plongée dans un échantillon de fluide (ou échantillon liquide).

Selon l'invention, des réactifs capables de se lier à l'analyte d'intérêt, en particulier des réactifs marqueurs capables de se lier à l'analyte d'intérêt, peuvent être positionnés en aval de la zone de dépôt d'échantillon ou peuvent être positionnés à l'intérieur de la zone de dépôt d'échantillon. En outre, la zone test est disposée en aval de la zone d'échantillon et contient des zones ou des lignes de test et de contrôle. La zone test contient un réactif ou une adaptation qui permet de retenir l'analyte d'intérêt dans la zone test. En particulier, le réactif ou l'adaptation inclus dans la zone test comprend un réactif de capture immobilisé qui se lie à l'analyte d'intérêt. Par conséquent, lorsque l'échantillon liquide s'écoule le long de la bandelette réactive, l'analyte d'intérêt se lie d'abord avec un réactif marqueur puis est retenu dans la zone test.

Avantageusement, selon l'invention, ledit support est composé d'un matériau biodégradable et/ou recyclable, comme par exemple du papier ou du carton.

De préférence, le dispositif de diagnostic selon l'invention est un dispositif de diagnostic à usage unique, ce qui signifie que le dispositif de diagnostic est généralement jeté après utilisation.

Préférentiellement, selon l'invention, ladite au moins une bandelette réactive est conçue pour détecter un analyte comprenant de la gonadotrophine chorionique (hCG).

Avantageusement, le dispositif de diagnostic selon l'invention comprend un logement situé à la face inférieure dudit support et étant agencé pour pouvoir y loger au moins une bandelette réactive. En particulier, un tel logement est agencé pour recevoir et garder en position stable au moins une bandelette réactive lors du test, sans pour autant qu'elle ne soit fixée dans le logement.

Préférentiellement, selon l'invention, ledit logement est agencé pour accueillir ladite au moins une bandelette réactive afin de la maintenir en place sans la fixer au support. Un tel maintien de ladite au moins une bandelette réactive dans le logement permet de garantir la réalisation d'un diagnostic fiable. Le logement peut par exemple se présenter sous la forme d'une cuvette dans laquelle ladite au moins une bandelette réactive est immobilisée sans toutefois y être fixée. Au sens de la présente invention, un tel logement peut être simplement défini entre la face supérieure et la face inférieure du support, ladite au moins une bandelette réactive étant alors maintenue entre ces deux faces sans pour autant être fixée au support.

Préférentiellement, selon l'invention, ladite zone de dépôt dudit échantillon liquide comprend un matériau absorbant en communication fluidique avec ladite au moins une bandelette réactive.

Selon un mode de réalisation suivant l'invention, ladite au moins une bandelette réactive est composée d'un matériau plastique et/ou d'un polymère tel que par exemple de la nitrocellulose.

Avantageusement, selon l'invention, ladite zone de rupture se présente sous la forme de perforations ou sous la forme d'un point de faiblesse prédéterminé ou sous la forme d'un système de fixation réversible ou sous la forme d'une ligne de rupture pré-imprimée ou sous la forme d'une charnière flexible ou sous la forme d'une zone de rupture adhésive ou sous la forme d'une zone de rupture à déverrouillage ou sous la forme d'une zone de rupture à déchirure assistée.

Avantageusement, le dispositif de diagnostic selon l'invention est fabriqué à partir de matériaux compatibles avec les normes de sécurité et de qualité pour les dispositifs médicaux.

Préférentiellement, le dispositif de diagnostic selon l'invention comprend une zone ou un élément de préhension situé dans un plan sensiblement parallèle au plan de la face supérieure ou de la face inférieure du support, ladite zone de préhension étant reliée au support par une zone ou un élément de liaison.

La présente invention porte également sur une utilisation d'un dispositif de diagnostic selon l'invention pour détecter et/ou identifier un analyte cible dans un échantillon liquide, en particulier pour détecter et/ou identifier la gonadotrophine chorionique (hCG).

D'autres caractéristiques, détails et avantages de l'invention ressortiront des exemples donnés ci-après, à titre non limitatif et en faisant référence aux figures annexées.

Les figures 1 à 4 illustrent un premier mode de réalisation d'un dispositif de diagnostic selon l'invention.

Les figures 5 à 8 illustrent un deuxième mode de réalisation d'un dispositif de diagnostic selon l'invention.

La figure 9 illustre un autre mode de réalisation d'un dispositif de diagnostic selon l'invention.

### Exemples

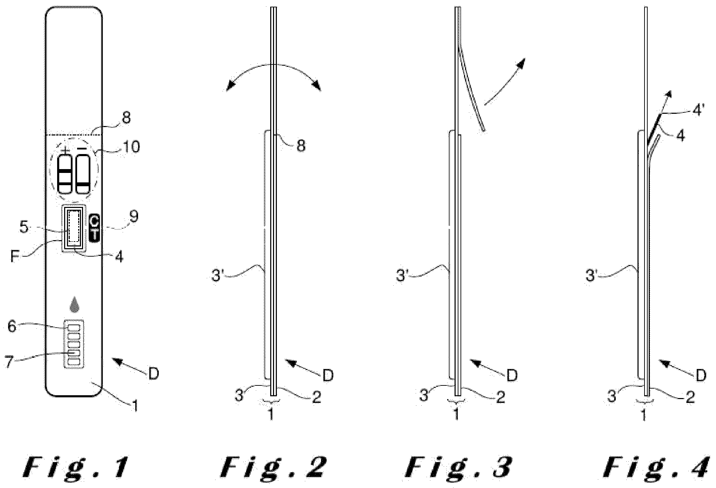

Les figures 1 à 4 illustrent un premier mode de réalisation d'un dispositif de diagnostic D selon l'invention. Selon ce premier mode de réalisation, le dispositif de diagnostic D pour détecter et/ou identifier un analyte cible dans un échantillon liquide comprend un support 1 présentant une face supérieure 2 et une face inférieure 3 et une bandelette réactive 4 renfermée à l'intérieur du support 1 entre sa face supérieure 2 et sa face inférieure 3. Selon ce mode de réalisation, la face inférieure 3 comprend un logement 3' agencé pour contenir et maintenir en position stable la bandelette réactive 4, sans toutefois fixer cette dernière au support 1 ou au logement 3'. Le support 1 est composé d'un matériau biodégradable et présente au moins une zone de rupture 8 agencée pour donner accès à un utilisateur à une extrémité libre 4' de ladite bandelette réactive 4. La bandelette réactive 4 comprend une zone test 5 et des réactifs permettant de détecter et/ou d'identifier ledit analyte cible. La face supérieure 2 du support 1 comprend des ouvertures 6 agencées pour donner accès à une zone de dépôt 7 de l'échantillon liquide, la zone de dépôt 7 étant en communication fluidique avec la zone test 5 de la bandelette réactive 4. La face supérieure 2 du support 1 comprend également une fenêtre de lecture F s'ouvrant sur la zone test 5 de la bandelette réactive 4 où a lieu la détection et/ou l'identification de l'analyte cible. Le dispositif de diagnostic tel qu'illustré comprend des impressions 9, 10 permettant à l'utilisateur d'interpréter les résultats s'affichant dans la fenêtre de lecture F.

Après utilisation du dispositif de diagnostic tel que décrit ci-dessus et illustré aux figures 1 à 4, l'utilisateur peut exercer un mouvement sur une partie du support 1 comme indiqué par la double flèche à la figure 2. Ce mouvement va permettre à la zone de rupture 8 de se rompre de telle sorte qu'une partie de la face supérieure 2 du support 1 puisse être aisément soulevée comme indiqué par la flèche à la figure 3. L'utilisateur a alors accès à une extrémité libre 4' de la bandelette réactive 4 contenue dans le logement 3' et peut extraire aisément la bandelette réactive 4 en saisissant son extrémité libre 4' comme indiqué par la flèche à la figure 4 de telle sorte à séparer le support 1 et la bandelette réactive 4.

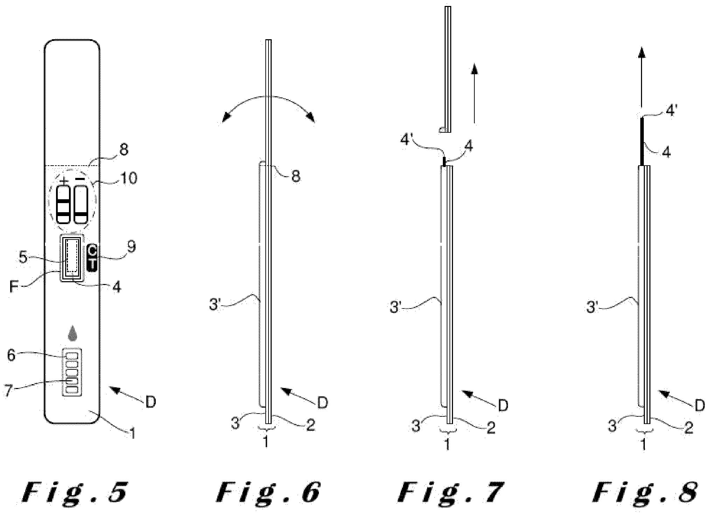

Les figures 5 à 8 illustrent un deuxième mode de réalisation d'un dispositif de diagnostic selon l'invention. Le dispositif de diagnostic D pour détecter et/ou identifier un analyte cible dans un échantillon liquide selon ce deuxième mode de réalisation comprend les mêmes éléments que ceux décrits pour le premier mode de réalisation, seule la zone de rupture 8 se présentant substantiellement sous une autre forme.

Après utilisation du dispositif de diagnostic D tel que décrit ci-dessus et illustré aux figures 5 à 8, l'utilisateur peut exercer un mouvement sur une partie du support 1 comme indiqué par la double flèche à la figure 6. Ce mouvement va permettre à la zone de rupture 8 de se rompre de telle sorte qu'une partie de la face supérieure 2 du support 1, qu'une partie de la face inférieure 3 du support 1 et qu'une partie du logement 3' se désolidarisent du reste du dispositif de diagnostic D par exemple par simple traction comme indiqué par la flèche à la figure 7. L'utilisateur a alors accès à l'extrémité libre 4' de la bandelette réactive 4 et peut extraire aisément la bandelette réactive 4 en attrapant / en saisissant son extrémité libre 4' comme indiqué par la flèche à la figure 8 de telle sorte à séparer le support 1 et la bandelette réactive 4.

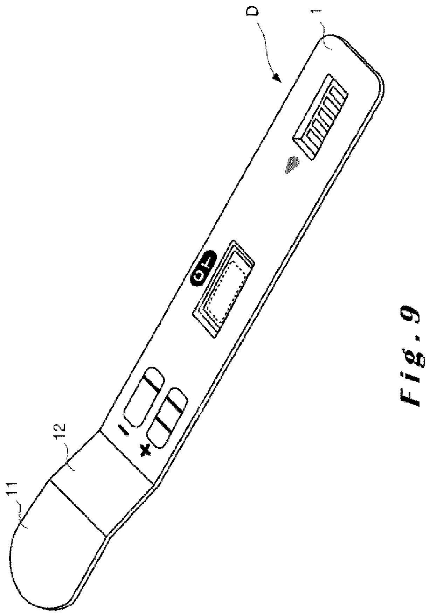

La figure 9 illustre un mode de réalisation d'un dispositif de diagnostic selon l'invention. Selon ce mode de réalisation, le dispositif de diagnostic comprend une zone ou un élément de préhension 11 situé dans un plan sensiblement parallèle au plan de la face supérieure 2 ou de la face inférieure 3 du support 1, la zone de préhension 11 étant reliée au support 1 par une zone ou un élément de liaison 12.

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Dispositif de diagnostic (D) pour détecter et/ou identifier un analyte cible dans un échantillon liquide, comprenant :
• un support (1) présentant une face supérieure (2) et une face inférieure (3), et
• au moins une bandelette réactive (4) renfermée à l'intérieur dudit support (1) entre ladite face supérieure (2) et ladite face inférieure (3), ladite au moins une bandelette réactive (4) comprenant une zone test (5) et des réactifs permettant de détecter et/ou d'identifier ledit analyte cible,
ladite face supérieure (2) dudit support (1) comprenant :
• au moins une ouverture (6) agencée pour donner accès à une zone de dépôt (7) dudit échantillon liquide, ladite zone de dépôt (7) étant en communication fluidique avec ladite zone test (5) de ladite au moins une bandelette réactive (4), et
• une fenêtre de lecture (F) s'ouvrant sur ladite zone test (5) de ladite au moins une bandelette réactive (4) où a lieu la détection et/ou l'identification dudit analyte cible,
ledit dispositif de diagnostic (D) étant **caractérisé en ce que** ledit support (1) présente au moins une zone de rupture (8) conçue pour donner accès à un utilisateur à une extrémité libre (4') de ladite au moins une bandelette réactive (4).

2. Dispositif de diagnostic (D) selon la revendication 1, **caractérisé en ce que** ledit support est composé d'un matériau biodégradable et/ou recyclable.

3. Dispositif de diagnostic (D) selon la revendication 1 ou 2, **caractérisé en ce que** qu'il est à usage unique.

4. Dispositif de diagnostic (D) selon l'une quelconque des revendications précédentes, **caractérisé en ce que** ladite au moins une bandelette réactive (4) est conçue pour détecter un analyte comprenant de la gonadotrophine chorionique (hCG).

5. Dispositif de diagnostic (D) selon l'une quelconque des revendications précédentes, **caractérisé en ce qu'**il comprend un logement (3') situé à la face inférieure (3) dudit support (1) et étant agencé pour pouvoir y loger ladite au moins une bandelette réactive (4).

6. Dispositif de diagnostic (D) selon l'une quelconque des revendications précédentes, **caractérisé en ce que** ladite zone de dépôt (7) dudit échantillon liquide comprend un matériau absorbant en communication fluidique avec ladite au moins une bandelette réactive.

7. Dispositif de diagnostic (D) selon l'une quelconque des revendications précédentes, **caractérisé en ce que** ladite au moins une bandelette réactive (4) est composée d'un matériau plastique et/ou d'un polymère tel que par exemple la nitrocellulose.

8. Dispositif de diagnostic (D) selon l'une quelconque des revendications précédentes, **caractérisé en ce que** ladite zone de rupture se présente sous la forme de perforations ou sous la forme d'un point de faiblesse prédéterminé ou sous la forme d'un système de fixation réversible ou sous la forme d'une ligne de rupture pré-imprimée ou sous la forme d'une charnière flexible ou sous la forme d'une zone de rupture adhésive ou sous la forme d'une zone de rupture à déverrouillage ou sous la forme d'une zone de rupture à déchirure assistée.

9. Dispositif de diagnostic (D) selon l'une quelconque des revendications précédentes, **caractérisé en ce qu'**il est fabriqué à partir de matériaux compatibles avec les normes de sécurité et de qualité pour les dispositifs médicaux.

10. Dispositif de diagnostic (D) selon l'une quelconque des revendications précédentes, **caractérisé en ce qu'**il comprend une zone ou un élément de préhension (11) situé dans un plan sensiblement parallèle au plan de la face supérieure (2) ou de la face inférieure (3) dudit support (1), ladite zone de préhension (11) étant reliée au support (1) par une zone ou un élément de liaison (12).

11. Utilisation d'un dispositif de diagnostic (D) selon l'une quelconque des revendications 1 à 10 pour détecter et/ou identifier un analyte cible dans un échantillon liquide, en particulier pour détecter et/ou identifier la gonadotrophine chorionique (hCG).

## Patentansprüche

1. Diagnosevorrichtung (D) zum Erkennen und/oder Identifizieren eines Zielanalyten in einer flüssigen Probe, umfassend:
- einen Träger (1), der eine obere Fläche (2) und eine untere Fläche (3) aufweist, und
- mindestens einen im Inneren des Trägers (1) zwischen der oberen Fläche (2) und der unteren Fläche (3) eingeschlossenen reaktiven Streifen (4), wobei der mindestens eine reaktive Streifen (4) einen Testbereich (5) und Reagenzien umfasst, die gestatten, den Zielanalyten zu erkennen und/oder zu identifizieren,
wobei die obere Fläche (2) des Trägers (1) Folgendes umfasst:
- mindestens eine Öffnung (6), die angeordnet ist, um Zugang zu einem Ablagebereich (7) der flüssigen Probe zu gewähren, wobei der Ablagebereich (7) in fluidischer Verbindung mit dem Testbereich (5) des mindestens einen reaktiven Streifens (4) vorliegt, und
- ein Lesefenster (F), das sich auf dem Testbereich (5) des mindestens einen reaktiven Streifens (4) öffnet, an dem die Erkennung und/oder Identifikation des Zielanalyten stattfindet,
wobei die Diagnosevorrichtung (D) **dadurch gekennzeichnet ist, dass** der Träger (1) mindestens eine Sollbruchstelle (8) aufweist, die konzipiert ist, um einem Anwender Zugang zu einem freien Ende (4') des mindestens einen reaktiven Streifens (4) zu gewähren.

2. Diagnosevorrichtung (D) nach Anspruch 1, **dadurch gekennzeichnet, dass** der Träger aus einem biologisch abbaubaren und/oder recycelbaren Material besteht.

3. Diagnosevorrichtung (D) nach Anspruch 1 oder 2, **dadurch gekennzeichnet, dass** sie zur einmaligen Verwendung bestimmt ist.

4. Diagnosevorrichtung (D) nach irgendeinem der vorstehenden Ansprüche, **dadurch gekennzeichnet, dass** der mindestens eine reaktive Streifen (4) zum Erkennen eines Analyten konzipiert ist, der humanes Choriongonadotropin (hCG) umfasst.

5. Diagnosevorrichtung (D) nach irgendeinem der vorstehenden Ansprüche, **dadurch gekennzeichnet, dass** sie eine Aufnahme (3') umfasst, die sich an der unteren Fläche (3) des Trägers (1) befindet und angeordnet ist, um darin den mindestens einen reaktiven Streifen (4) unterzubringen.

6. Diagnosevorrichtung (D) nach irgendeinem der vorstehenden Ansprüche, **dadurch gekennzeichnet, dass** der Ablagebereich (7) der flüssigen Probe ein Absorptionsmaterial in fluidischer Verbindung mit dem mindestens einen reaktiven Streifen umfasst.

7. Diagnosevorrichtung (D) nach irgendeinem der vorstehenden Ansprüche, **dadurch gekennzeichnet, dass** der mindestens eine reaktive Streifen (4) aus einem Kunststoffmaterial und/oder einem Polymer wie zum Beispiel Nitrocellulose besteht.

8. Diagnosevorrichtung (D) nach irgendeinem der vorstehenden Ansprüche, **dadurch gekennzeichnet, dass** die Sollbruchstelle in Form von Perforationen oder in Form eines vorbestimmten Schwachpunkts oder in Form eines Systems zur reversiblen Befestigung oder in Form einer vorgedruckten Bruchlinie oder in Form eines flexiblen Scharniers oder in Form einer Klebesollbruchstelle oder in Form einer Entriegelungs-Sollbruchstelle oder in Form einer Sollbruchstelle mit unterstützter Einrissbildung vorliegt.

9. Diagnosevorrichtung (D) nach irgendeinem der vorstehenden Ansprüche, **dadurch gekennzeichnet, dass** sie ausgehend von mit den Sicherheits- und Qualitätsnormen für medizinische Vorrichtungen kompatiblen Materialien angefertigt ist.

10. Diagnosevorrichtung (D) nach irgendeinem der vorstehenden Ansprüche, **dadurch gekennzeichnet, dass** sie ein(en) Griffbereich oder -element (11) umfasst, der/das sich in einer Ebene im Wesentlichen parallel zu der Ebene der oberen Fläche (2) oder der unteren Fläche (3) des Trägers (1) befindet, wobei der Griffbereich (11) durch ein(en) Verbindungsbereich oder -element (12) mit dem Träger (1) verbunden ist.

11. Verwendung der Diagnosevorrichtung (D) nach irgendeinem der Ansprüche 1 bis 10 zum Erkennen und/oder Identifizieren eines Zielanalyten in einer flüssigen Probe, insbesondere zum Erkennen und/oder Identifizieren des humanen Choriongonadotropins (hCG).

## Claims

1. A diagnostic device (D) for detecting and/or identifying a target analyte in a liquid sample, comprising:
• a support (1) with an upper face (2) and a lower face (3), and
• at least one reagent strip (4) enclosed inside said support (1) between said upper face (2) and said lower face (3), said at least one reagent strip (4) comprising a test zone (5) and reagents enabling said target analyte to be detected and/or identified,
said upper face (2) of said support (1) comprising:
• at least one opening (6) designed to give access to a zone (7) for depositing said liquid sample, said deposition zone (7) being in fluid communication with said test zone (5) of said at least one reagent strip (4), and
• a reading window (F) opening onto said test zone (5) of said at least one reagent strip (4) where said target analyte is detected and/or identified,
said diagnostic device (D) being **characterised in that** said support (1) has at least one rupture zone (8) designed to give a user access to a free end (4') of said at least one reagent strip (4).

2. The diagnostic device (D) as claimed in claim 1, **characterised in that** said support is made of a biodegradable and/or recyclable material.

3. The diagnostic device (D) according to claim 1 or 2, **characterised in that** it is for single use only.

4. The diagnostic device (D) according to any one of the preceding claims, **characterised in that** said at least one reagent strip (4) is designed to detect an analyte comprising chorionic gonadotropin (hCG).

5. The diagnostic device (D) according to any one of the preceding claims, **characterised in that** it comprises a housing (3') located on the lower face (3) of said support (1) and being arranged to accommodate said at least one reagent strip (4).

6. The diagnostic device (D) according to any one of the preceding claims, **characterised in that** said zone (7) for depositing said liquid sample comprises an absorbent material in fluid communication with said at least one reagent strip.

7. The diagnostic device (D) according to any one of the preceding claims, **characterised in that** said at least one reagent strip (4) is made of a plastic material and/or a polymer such as nitrocellulose, for example.

8. The diagnostic device (D) according to any one of the preceding claims, **characterised in that** said rupture zone is in the form of perforations or in the form of a predetermined point of weakness or in the form of a reversible fastening system or in the form of a preprinted rupture line or in the form of a flexible hinge or in the form of an adhesive rupture zone or in the form of a release rupture zone or in the form of an assisted tear rupture zone.

9. The diagnostic device (D) according to any one of the preceding claims, **characterised in that** it is manufactured from materials compatible with safety and quality standards for medical devices.

10. The diagnostic device (D) according to any one of the preceding claims, **characterised in that** it comprises a gripping zone or element (11) situated in a plane substantially parallel to the plane of the upper face (2) or lower face (3) of said support (1), said gripping zone (11) being connected to the support (1) by a connecting zone or element (12).

11. A use of a diagnostic device (D) according to any one of claims 1 to 10 for detecting and/or identifying a target analyte in a liquid sample, in particular for detecting and/or identifying chorionic gonadotropin (hCG).
